(19) Europäisches Patentamt
European Patent Office
Office européen des brevets

(11) Publication number: 0 427 925 A2

# (12) EUROPEAN PATENT APPLICATION

(21) Application number: 90115326.2

(51) Int. Cl.5: A61K 31/415, A61K 31/675

(22) Date of filing: 09.08.90

The title of the invention has been amended (Guidelines for Examination in the EPO, A-III, 7.3).

(30) Priority: 10.08.89 US 392238
25.06.90 US 541071

(43) Date of publication of application:
22.05.91 Bulletin 91/21

(84) Designated Contracting States:
AT BE CH DE DK ES FR GB GR IT LI LU NL SE

(71) Applicant: WARNER-LAMBERT COMPANY
201 Tabor Road
Morris Plains New Jersey 07950(US)

(72) Inventor: Boxer, Peter Alan
1356 Glenleven
Ann Arbor, Michigan 48103(US)
Inventor: Wallace, Jan David
2510 Craig
Ann Arbor, Michigan 48103(US)

(74) Representative: Mansmann, Ivo
c/o Gödecke AG - Patentabteilung Postfach
569 Mooswaldallee 1-9
W-7800 Freiburg(DE)

## (54) Use of prophenytoin for preparing pharmaceutical preparations for treating stroke and cerebral ischemia.

(57) The present invention relates to a new therapeutic indication of known derivatives of phenytoin or a stable pharmaceutical composition of the phenytoin prodrug which is now known as fosphenytoin sodium. More particularly, the present invention concerns a method of preparing pharmaceutical compositions for treating cerebral ischemia or stroke in a human by using the derivatives of phenytoin, preferably 3-(hydroxy-methyl)-5,5-diphenylhydantoin phosphate ester disodium salt or a prodrug of phenytoin as active compounds. The preferred compound is also variously known as the fosphenytoin sodium; 5,5-diphenyl-3-[(phosphonooxy)methyl]-2,4-imidazolidinedione disodium salt; 3-phosphoryloxymethyl-5,5-diphenylhydantoin disodium salt; or 3-(hydroxymethyl)-5,5-diphenylhydantoin disodium phosphate ester

EP 0 427 925 A2

# USE OF PROPHENYTOIN FOR TREATING STROKE

## BACKGROUND OF THE INVENTION

### 1. Field of the Invention

The present invention relates to a novel therapeutic indication of known derivatives of phenytoin and stable compositions thereof. More particularly, the present invention concerns a method of preparing pharmaceutical compositions useful for treating stroke in a human at risk of or suffering from stroke, as defined hereinafter. The invention comprises the use of derivatives of phenytoin, preferably 3-(hydroxy-methyl)-5,5-diphenylhydantoin phosphate ester disodium salt, a prodrug of phenytoin and most preferably 3-(hydroxy-methyl)-5,5-diphenylhydantoin disodium phosphate ester for the preparation of pharmaceutical compositions. This compound is also variously known as fosphenytoin sodium; the disodium salt of prophenytoin; 5,5-diphenyl-3-[(phosphonooxy)methyl]-2,4-imidazolidinedione disodium salt; 3-phosphoryloxymethyl-5,5-diphenylhydantoin disodium salt; or 3-(hydroxymethyl)-5,5-diphenylhydantoin disodium phosphate ester.

### 2. Description of the Prior Art

The known derivatives of phenytoin include the 5,5-diphenylhydantoins or salts thereof disclosed in U.S. Patent No. 4,260,769. These are the prophenytoins for use in the present invention. U.S. Patent No. 4,260,769 is, therefore, incorporated by reference. The only uses disclosed in U.S. Patent No. 4,260,769 are as anticonvulsants, antiepileptics, and antiarrhythmics. Stable pharmaceutical compositions of 3-(hydroxymethyl)-5,5-diphenylhydantoin disodium phosphate ester are disclosed in U.S. Patent No. 4,925,860, which is hereby incorporated by reference for these compositions to be used in the method of this invention. The references in the Background of the Invention of U.S. Patent No. 4,925,860 are also incorporated by reference.

Specifically the prophenytoins of U.S. Patent No. 4,260,769 are compounds having the structural formula (I)

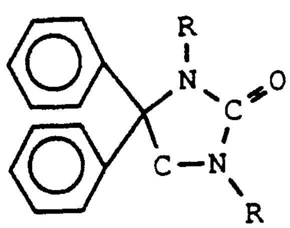

I

or the pharmaceutically acceptable acid addition or base salt, $C_1$-$C_4$ alkylhalide quaternary salt or N-oxide thereof; wherein each R is hydrogen or $-CH(R_1)-X-P(O)-(OH)_2$ wherein $R_1$ is hydrogen or $C_1$-$C_7$ straight or branched chain alkyl, and X is O or S, with the proviso that both R's cannot simultaneously be hydrogen.

Further, the preferred method of use is for the compound 5,5-diphenylhydantoin of the formula I or its pharmaceutically acceptable acid addition or base salt and it is particularly now found to have a novel use for preparing pharmaceutical compositions useful for treating stroke. A more preferred compound of the present method of use invention is the 3-phosphoryloxymethyl-5,5-diphenylhydantoin and a most preferred compound of the present method of use invention is the disodium salt thereof or fosphenytoin sodium.

The pharmaceuticals may be used for treating stroke in a mammal particularly a human, suffering from or susceptible to stroke in need of such treatment by administering the above-noted stable pharmaceutical composition of 3-(hydroxymethyl)-5,5-diphenylhydantoin disodium phosphate ester, preferably in unit dosage form.

Numerous references disclose protective effects of phenytoin in cerebral ischemia of which the following are merely representative examples:

J. P. Cullen, et al, Protective Action of Phenytoin in Cerebral Ischemia", Anesthesia and Analgesia, Vol. 58,

No. 3, pp. 165-169 (May-June 1979);
A. A. Artru and J. D. Michenfelder, "Anoxic Cerebral Potassium Accumulation Reduced by Phenytoin: Mechanism of Cerebral Protection?", Anesthesia and Analgesia, Vol. 60, No. 1, pp. 41-45 (January 1981);
J. Antonio Aldrete, "Phenytoin in the Treatment of Cerebral Ischemia", Anesthesia and Analgesia, Vol. 60, No. 8, p. 616 (August 1981);
G. K. Shiu, et al, "Dose of Thiopental, Pentobarbital, and Phenytoin for Maximal Therapeutic Effects in Cerebral Ischemic Anoxia," Critical Care Medicine, Vol. 11, No. 6, pp. 452-459 (1983);
A. Fukuda, H. Tabuse, N. Ihara, J. Tanabe, H. Ikeda, and A. Kohama, "Effects of Phenytoin on Regional Cerebral Blood Flow, Electroencephalogram, and Electrolyte Contents in Cerebral Blood and Cerebral Cortex Following Total Cerebral Ischemia in Dogs", Circulatory Shock, Vol. 11, pp. 341-350 (1983);
J. Suzuki, H. Abiko, K. Mizoi, M. Oba, and T. Yoshimoto, "Protective Effect of Phenytoin and its Enhanced Action by Combined Administration with Mannitol and Vitamin E in Cerebral Ischemia", Acta Neurochir. - (Wien) Vol. 88, pp. 56-64 (1987);
R. Massei, E. DeSilva, B. R. Rabbiati, L. Infuso, L. Rovagnati, and C. A. Altamura, "Cerebral Protection with Diphenylhydantoin During Disobligating Surgery of the Supra-Aortic Branches," J. Neurosurg. Vol. 27, pp. 107-110 (1983) and
Fukuda, A, et al discusses "Protective Effects of Anticonvulsant Drugs Against Complete Cerebral Ischemia in Mice," Igaku to Seibutsugaku, Vol. 118, No. 2, pp. 85-87 (1989). See CA: 110(19)166 017a.

Furthermore, numerous reports are available disclosing the advantages of the known derivatives of phenytoin, including the phosphate ester of 3-hydroxymethyl-5,5-diphenylhydantoin or its disodium salt as a prodrug of phenytoin with advantageous physicochemical properties. For example, see the following:
U.S. Patent No. 4,260,769 noted above; S. A. Varia, et al, "Phenytoin Prodrugs III: Water-Soluble Prodrugs for Oral and/or Parenteral Use", J. of Pharmaceutical Sciences, Vol. 73, No. 8, pp. 1068-1073 (August 1984);
R. D. Smityh, et al, "Pharmacology of ACC-9653 (Phenytoin Prodrug)," Epilepsia, Vol. 30, Suppl. 2, pp. 15-20;
I. E. Leppik, et al, "Phenytoin Prodrug: Preclinical and Clinical Studies," Epilepsia, Vol. 30, Suppl. 2, pp. 22-26; and
T. R. Browne, et al, "Bioavailability of ACC-9653 (Phenytoin Prodrug), Epilepsia, Vol. 30, Suppl. 2, pp. 27-32.

However, there is no disclosure in the references noted above to make obvious the present invention for a novel use of the 5,5-diphenylhydantoins of U.S. 4,260,769 to treat stroke.

## SUMMARY OF THE INVENTION

Accordingly, the present invention provides a method of preparing pharmaceutical compositions useful for treating stroke as defined hereinafter in a human in need of such treatment, such as at risk of or suffering from the stroke. The active compound is a prophenytoin which is a compound of the formula (I)

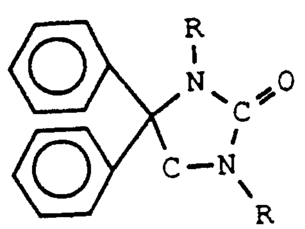

I

or the pharmaceutically acceptable acid addition or basic salt, $C_1$-$C_4$ alkylhalide quaternary salt or N-oxide thereof;
wherein each R is independently selected from the group consisting of hydrogen and $-CH(R_1)-O-P-(O)(OH)_2$ wherein $R_1$ is selected from the group consisting of hydrogen and $C_{1-7}$ straight or branched chain alkyl; with the proviso that the R's cannot simultaneously be hydrogen.

The present invention also provides a method of preparing pharmaceutical compositions useful for treating stroke as defined hereinafter for one, preferably a human suffering from or susceptible to stroke in need of such treatment. The pharmaceutical consists of a stable pharmaceutical composition which is the prodrug 3-(hydroxymethyl)-5,5-diphenylhydantoin disodium phosphate ester (hereinafter the phenytoin prodrug) in an aqueous system maintained at a pH of about 8.3 to 9.4 to produce diphenylglycinamide as the primary degradant with minimal quantities of phenytoin; preferably having 35 to about 180 mg, more preferably having 35 to 130 mg or most preferably about 75 mg of composition prodrug/mL, and 0.05 to 0.2 M buffer.

The stable pharmaceutical composition is preferably an injectable, aqueous pharmaceutical composition for the treatment of stroke or cerebral ischemia as defined hereinafter comprising an effective amount of 3-(hydroxymethyl)-5,5-diphenylhydantoin disodium phosphate ester or fosphenytoin sodium (phenytoin prodrug) for treating such stroke or ischemic states, said composition comprising about 35 mg to about 180 mg of prodrug/mL of solution and 0.05 to 0.2 M buffer; said composition having a pH range of about 8.3 to 9.4, the prodrug degrading in aqueous solution to prodruce diphenylglycinamide as the primary degradant with minimal quantities of phenytoin, the pH range of said composition acting to allow the degradation to proceed whereby the shelf-life of the prodrug in an aqueous composition is maximized.

The present invention most preferably also provides for a method of preparing pharmaceutical compositions useful for the treatment of a stroke as defined hereinafter in a mammal, particularly human, where the composition is administered by subcutaneous, intramuscular, or intravenous route to a mammal in need of such treatment an antistroke amount of a stable, pharmaceutical composition of the prodrug 3-(hydroxy-methyl)-5,5-diphenylhydantoin disodium phosphase ester or fosphenytoin sodium, said composition comprising from about 35 to 130 mg/mL of prodrug or fosphenytoin sodium, and from about 0.05 to 2 M of a buffer effective to maintain a pH of about 8.3 to 9.4, the prodrug degrading in aqueous solution to produce diphenylglycinamide as the primary degradant with minimal quantities of phenytoin, the pH range of said composition acting to allow the degradation to proceed whereby the shelf-life of the prodrug in an aqueous composition is maximized.

The present invention, however, relates to the discovery that the formula I or its pharmaceutically acceptable acid addition or base salt as defined above, particularly fosphenytoin sodium, has activity for a novel method of use specifically for treating subjects susceptible to or suffering from strokes. The stroke as referred to in the present invention is a cerebral vascular disease and may also be referred to as a cerebral vascular accident (CVA) and specifically includes acute thromboembolic stroke. Also included in cerebral vascular disease are transient cerebral ischemic attacks and other cerebral vascular problems accompanied by cerebral ischemia. Particularly, a subject susceptible to such strokes for the practice of the present invention may be the subject undergoing carotid endarterectomy, specifically, or other cerebrovascular or vascular surgical procedures, in general, or diagnostic vascular procedures including cerebral angiography in some cases, and the like. Other incidents which may be included among those for which the present method may be applied are head trauma, spinal cord trauma, or brain injury from general anoxia, hypoxia, hypoglycemia, hypotension as well as similar injuries seen during some procedure from embole, hypoperfusion, and hypoxia. An ordinary skilled physician would be able to determine the appropriate situation in which subjects susceptible to or at risk of stroke as well as suffering from stroke for administration by the methods of the present invention.

Particular incidents in a subject susceptible to stroke or in need of antistroke treatment may include, for example, cardiac bypass surgery, intracranial hemorrhage, perinatal asphyxia, cardiac arrest, and status epilepticus.

Subjects as used herein are mammals, including humans.

## DETAILED DESCRIPTION OF THE INVENTION

According to this invention, formula I or its pharmaceutically acceptable acid addition or base salt and particularly fosphenytoin sodium or the above-noted stable pharmaceutical composition, which is an agent for treating a subject susceptible to stroke or suffering from stroke as defined herein, may be administered in an effective amount which comprises a total intravenous or intramuscular injection of the prophenytoin of the formula I or its pharmaceutically acceptable acid addition or base salt or the above-noted stable pharmaceutical composition, preferably of fosphenytoin sodium. Such amount ranges from an amount initially administered before stroke in a subject at risk or up to 24 hours from the onset of the stroke, preferably as soon as possible following onset of stroke. The use may include administration of a subsequent amount for as long as 2 weeks following initial administration.

A preferred method of use comprises the compound of the formula I above wherein either R is hydrogen with the other being $-CH(R_1)-O-P(O)(OH)_2$ or a basic salt thereof and more preferably provides the compound is 3-(hydroxymethyl)-5,5-diphenylhydantoin phosphate ester or its disodium salt.

A more preferred method of use provides the preparation of a stable pharmaceutical composition which is an injectable, aqueous pharmaceutical composition for the treatment of stroke or ischemic states comprising an effective amount of 3-(hydroxymethyl)-5,5-diphenylhydantoin disodium phosphate ester (phenytoin prodrug) for treating such stroke or ischemic states, said composition comprising about 35 mg to about 180 mg of prodrug/mL of solution and 0.05 to 0.2 M buffer; said composition having a pH range of about 8.3 to 9.4, the prodrug degrading in aqueous solution to produce diphenylglycinamide as the primary degradant with minimal quantities of phenytoin, the pH range of said composition acting to allow the degradation to proceed whereby the shelf-life of the prodrug in an aqueous composition is maximized.

A preferred example of the stable pharmaceutical composition would contain:

composition prodrug: 35 to 180 mg/mL
alcohol: USP 0% to 25%
propylene glycol: 0% to 25%
L-arginine: 0 to 0.2 M
sodium desoxycholate: 0 to 0.1 M
polysorbate-80: 0% to 1.5%
tromethamine: 0.05 to 0.2 M

in water for injection with the pH adjusted from 8 to 10 with hydrochloric acid or sodium hydroxide.

The pH range found to provide the greatest stability is a pH of about 8.3 to 9.4. In this pH range, the choice of buffers is limited, namely, to buffers effective to maintain a pH of about 8 to 10. In addition to tromethamine, tris-(hydroxymethyl)aminomethane; other buffers which can be used are bicine, N,N-bis(2-hydroxyethyl)-2-aminoethanesulfonic acid; tricine, N-[tris(hydroxymethyl)methyl]glycine; sodium bicarbonate; glycylglycine; Hepes, N-2-hydroxyethylpiperazine-N′-2-ethanesulfonic acid; Hepps, N-2-hydroxyethylpiperazine-N'3-propanesulfonicacid; sodium phosphate; and Taps, 3[[tris(hydroxymethyl)-methyl]amino]propanesulfonic acid. Tromethamine, bicine or tricine are preferred. Suitable wetting agents in addition to polysorbate-80 are: polyoxamer 188 and polyoxyethylene fatty acid esters. A more specific composition would be 75 mg/mL prodrug; 0.1 M tromethamine and a pH of about 8.3 to 9.

The following tables illustrate the stability of a composition of the phenytoin prodrug, preferably fosphenytoin sodium, comprising 75.7 mg/mL composition prodrug, 0.1 M tromethamine, pH adjusted to 9.1 with HCl.

TABLE I.

| Mass Balance of Formaldehyde in Degraded Prodrug | | | | | | | | |
|---|---|---|---|---|---|---|---|---|
| Lot | Age (Mo) | Temp | pH | Initial Potency | Current Potency | Percent as Composition Prodrug | | Percent Recovery |
| | | | | | | DIZ | Form | |
| 2131 | 27 | RT | 8.9 | 98.22 | 93.75 | 0.196 | 5.553 | 101.30 |
| 2140 | 27 | RT | 8.9 | 96.42 | 92.13 | 0.172 | 5.042 | 100.96 |
| 2183 | 24 | RT | 8.8 | 98.06 | 94.30 | 0.130 | 4.450 | 100.84 |
| 2183 | 24 | RT | 8.8 | 98.06 | 94.30 | 0.135 | 4.314 | 100.70 |
| 2211 | 23 | RT | 8.3 | 104.52 | 103.38 | 0.033 | 1.509 | 100.38 |
| 2224 | 23 | RT | 8.9 | 103.75 | 97.24 | 0.155 | 4.349 | 98.07 |
| 2293 | 16 | RT | 8.5 | 100.91 | 99.11 | 0.055 | 1.747 | 100.00 |
| 2295 | 16 | RT | 8.3 | 101.49 | 99.04 | 0.040 | 1.303 | 98.91 |
| 2327 | 9 | 40 | 8.9 | 100.42 | 93.74 | 0.382 | 7.893 | 101.59 |
| 2327 | 8 | 40 | 8.9 | 101.41 | 94.88 | 0.342 | 7.450 | 101.24 |
| 2358 | 7 | 40 | 8.9 | 98.70 | 94.34 | 0.251 | 6.653 | 102.58 |
| 2360 | 7 | 40 | 8.9 | 99.63 | 94.61 | 0.246 | 5.364 | 100.59 |
| DIZ = 5,5-diphenyl-4-imidazolidinone<br>Form = formaldehyde | | | | | | | | |

TABLE II.

| Mass Balance of the 75 mg/mL Compositions Prodrug Using Method I | | | | | | | | | | | | |
|---|---|---|---|---|---|---|---|---|---|---|---|---|
| Lot | Age (Mo) | Temp | pH | Initial Potency | Potency | Degradation Products (Percent as Composition Prodrug) | | | | | | Percent Recovery |
| | | | | | | Phen | DPG | BZP | DIZ | UNKB | Form | |
| 2131 | 27 | RT | 8.8 | 98.22 | 93.75 | 0.211 | 0.295 | 0.001 | 0.196 | 5.046 | 5.553 | 101.30 |
| 2140 | 27 | RT | 8.8 | 96.42 | 92.13 | 0.189 | 0.245 | 0.004 | 0.172 | 4.604 | 5.042 | 100.96 |
| 2183 | 24 | RT | 8.8 | 98.06 | 94.30 | 0.131 | 0.053 | 0.002 | 0.130 | 4.264 | 4.450 | 100.84 |
| 2183 | 24 | RT | 8.8 | 98.06 | 95.57 | 0.130 | 0.049 | 0.003 | 0.135 | 4.132 | 4.314 | 102.00 |
| 2211 | 23 | RT | 8.3 | 104.52 | 103.38 | 1.016 | 0.011 | 0.0003 | 0.033 | 0.482 | 1.509 | 100.38 |
| 2224 | 23 | RT | 8.9 | 103.75 | 97.24 | 0.106 | 0.047 | 0.0015 | 0.155 | 4.195 | 4.349 | 98.07 |
| 2293 | 16 | RT | 8.5 | 100.91 | 99.11 | 0.053 | 0.000 | 0.0005 | 0.055 | 1.694 | 1.747 | 100.00 |
| 2295 | 16 | RT | 8.3 | 101.49 | 99.04 | 0.059 | 0.000 | 0.0006 | 0.040 | 1.243 | 1.303 | 98.91 |
| 2327 | 9 | 40 | 8.8 | 100.42 | 93.74 | 1.485 | 1.147 | 0.013 | 0.382 | 5.248 | 7.893 | 101.59 |
| 2327 | 8 | 40 | 8.8 | 101.41 | 94.88 | 1.071 | 0.746 | 0.014 | 0.342 | 5.619 | 7.450 | 101.24 |
| 2358 | 7 | 40 | 8.8 | 98.70 | 94.34 | 0.769 | 0.404 | 0.006 | 0.251 | 5.474 | 6.653 | 102.58 |
| 2360 | 7 | 40 | 8.8 | 99.63 | 94.61 | 0.800 | 0.419 | 0.005 | 0.246 | 4.140 | 5.374 | 100.59 |
| | | | | | | | | | Average Recovery | | | 100.71 |

PHEN = Phenytoin

DPG = Diphenylglycine

BZP = Benzophenone

DIZ = 5,5-diphenyl-4-imidazolidinone

UNKB* = Unknown B, calculated as follows: UNKB = FORM-PHEN-DGP-BZP

FORM = Formaldehyde

NOTE: Unknown B (UNKB) has been identified as diphenylglycinamide

TABLE III.

| New Mass Balance of 75 mg/mL Composition Prodrug Using Method II | | | | | | | | | |
|---|---|---|---|---|---|---|---|---|---|
| Lot | Age (Mo) | Temp | Age | 9653 Percent of Initial | Degradation Products (Percent as Composition Prodrug) | | | | Percent Recovery |
| | | | | | PHEN | DPG | DIZ | DPGA | |
| 2131 | 30 | RT | 8.8 | 93.06 | 0.141 | 0.243 | 0.174 | 5.144 | 98.75 |
| 2140 | 30 | RT | 8.8 | 94.79 | 0.148 | 0.247 | 0.182 | 5.240 | 100.61 |
| 2183 | 27 | RT | 8.8 | 98.40 | ND | 0.116 | 0.137 | 3.021 | 101.67 |
| 2211 | 25 | RT | 8.3 | 98.64 | 0.097 | 0.041 | 0.027 | 1.254 | 100.06 |
| 2224 | 24 | RT | 8.8 | 94.85 | 0.104 | 0.110 | 0.163 | 3.810 | 99.04 |
| 2293 | 18 | RT | 8.5 | 98.70 | ND | 0.080 | ND | 1.631 | 100.41 |
| 2295 | 18 | RT | 8.3 | 98.53 | ND | 0.032 | 0.034 | 1.163 | 99.76 |
| 2327 | 10 | 40 | 8.8 | 92.11 | 1.491 | 1.224 | 0.437 | 5.068 | 100.33 |
| 2358 | 9 | 40 | 8.8 | 93.21 | 1.110 | 0.757 | 0.329 | 4.153 | 99.56 |
| 2360 | 9 | 40 | 8.8 | 92.34 | 1.157 | 0.766 | 0.323 | 4.115 | 98.70 |
| | | | | | | Average Recovery | | | 99.89 |
| PHEN = Phenytoin<br>DPG = Diphenylglycine<br>DIZ = 5,5-Diphenyl-4-imidazolidinone<br>DPGA = Diphenylglycinamide (UNKB in Table II)<br>ND = None detected | | | | | | | | | |

Initially, it was proposed that the composition prodrug decomposed via a simple two-step pathway resulting in phenytoin. However, it has been determined that phenytoin is not the only decomposition product and could only account for a small portion of the degraded composition prodrug. The degradation pathway has been determined to proceed from phenytoin prodrug to formaldehyde, 5,5-diphenyl-4-imidazolidinone (DIZ), diphenylglycinamide, diphenylglycine, benzophenone, and phenytoin via the pathways described.

## Overall Degradation Pathway(s)

The overall degradation pathway is shown in Scheme V. Many of the steps involved include consumption of hydroxyl, indicating that the pH of the formulated product should decrease with time. This has been observed in the routine stability program for this product. The addition of a buffer to prevent the pH from dropping significantly from its initial value over the lifetime of the product is required. Small changes in the degradation pathway occur as the pH drops. It is seen that as the pH drops the rate of phenytoin formation increases and the rate of DIZ formation decreases. This indicates that the proposed hydantoin ring opening by the phosphate appears to be the rate limiting step in the primary degradation pathway of composition prodrug. However, formation of phenytoin increases and the solubility of phenytoin decreases as the pH is lowered thus, diminishing the shelf-life of the product due to the saturation of the aqueous solution and eventual precipitation of phenytoin. The chosen pH of the finished product allows the degradation to proceed such that the primary degradant is diphenylglycinamide, while minimal quantities of phenytoin are produced and shelf-life is maximized.

## Mass Balance Determination

Several samples at room temperature and 40° C were analyzed to determine mass balance. Analysis of phenytoin prodrug potency, formaldehyde, 2,2-diphenylglycine, 5,5-diphenylhydantoin, 5,5-diphenyl-4-imidazolidinone, and benzophenone concentrations were performed using the gradient HPLC method. Analysis

of phenytoin prodrug potency, 2,2-diphenylglycine, 2,2-diphenylglycinamide (as 2,2-diphenylglycine), 5,5-diphenylhydantoin, and 5,5-diphenyl-4-imidazolidinone concentrations were determined using the isocratic HPLC method. Molar equivalents were calculated and mass balance results were determined and compared.

## Method Statistics and Comparison

### Method I - Gradient Method

Several standard curves of each compound were chromatographed for the determination of method linearity. The peak areas and heights were plotted versus respective concentrations to yield calibration curves. All standard curves had a correlation coefficient of >0.99. Standard peak heights or areas were shown to be within 4% of the linear regression line thereby passing preestablished system suitability requirements. Replicate injections of a standard solution of each compound were chromatographed. Relative standard deviations were less than 1.7% using peak areas and less than 2.2% using peak heights. These results confirm the method is precise.

The same dilutions of standards were used for determination of limit of detection and limit of quantitation.

All standards were chromatographed. Peak areas were measured by the data system and linearity was confirmed. Data from standard curve injections before and after a set of sample injections were collected to determine the suitability and effectiveness of the HPLC system.

Limits of quantitation were experimentally determined and are shown below.

| | Peak Area | Peak Height |
|---|---|---|
| Diphenylglycine | 8.3 µg/mL | 27.7 µg/mL |
| Diphenylhydantoin | 0.5 µg/mL | 3.0 µg/mL |
| Diphenyl-4-imidazolidinone | 8.9 µg/mL | 1.5 µ/mL |
| Benzophenone | 0.05 µg/mL | 0.05 µg/mL |

Limits of detection were experimentally determined and are shown below.

| | Peak Area or Height |
|---|---|
| Diphenylglycine | 1.0 µg/mL |
| Diphenylhydantoin | 0.3 µg/mL |
| Diphenyl-4-imidazolidinone | 0.5 µg/mL |
| Benzophenone | 0.01 µg/mL |

A combination standard curve containing 2,2-diphenylglycine, 5,5-diphenylhydantoin, and 5,5-diphenyl-4-imidazolidinone was chromatographed several times for the determination of equality with individual standard curves. The peak areas and heights were plotted versus respective concentrations to yield calibration curves. Slopes and y-intercepts were compared to individual standards, confirming their equality. Replicate injections of a combination standard solution were chromatographed. Relative standard deviations were less than 1.7% using peak areas and less than 2.0% using peak heights with the exception of diphenylglycine, which was 3.09% using peak heights. This confirms the method is precise using the combination standard curve.

### Mass Balance

Several samples kept at various lengths of time at room temperature and 40 °C were analyzed to determine mass balance. Initial and current potency, along with current formaldehyde concentration data, were used to determine mass balance. Diphenylglycine, diphenylhydantoin, diphenyl-4-imidazolidinone, and benzophenone concentrations were chromatographically determined using Method I. From this data, mass balance was calculated. Initial and current potency values were converted to initial and current moles of phenytoin prodrug. Formaldehyde, diphenylglycine, diphenylhydantoin, diphenyl-4-imidazolidinone, and benzophenone concentrations were converted to molar equivalents. Using the value of moles of formaldehyde, diphenylglycine, diphenylhydantoin, and benzophenone, the moles of Unknown B (which later was determined to be diphenylglycinamide) were calculated using Equation 1.

Unknown B = Formaldehyde (moles) - diphenylglycine (moles) diphenylhydantoin (moles) - benzophenone (moles) (1)

Moles Determined = Current moles + Unknown B (moles)* + diphenylglycine (moles) + diphenylhydantoin (moles) + diphenyl-4-imidazolidinone (moles) + benzophenone (moles) (2)

$$\%\ \text{of Initial Recovered} = \frac{\text{Moles Determined}}{\text{Initial Moles}} \times 100 \qquad (3)$$

* Unknown B was determined to be diphenylglycinamide

Method II - Isocratic Method Linearity

Standard curves were prepared and injected to determine the linearity of the isocratic method. In each case, linear regression lines for diphenylglycine, 5,5-diphenyl-4-imidazolidinone, and phenytoin had correlation coefficients of greater than 0.999. Diphenylglycinamide was identified as a compound of degradation, previously referred to as Unknown B. As no standard for diphenylglycinamide is available, the curve for diphenylglycine was used to determine the concentrations of the samples.

Precision of the isocratic method was performed only by analysis of several lots of degraded prodrug and comparing the results to the results of the gradient method (see Tables II and III). The results showed very favorable comparisons for all the degradants. The major difference is seen in the diphenylglycinamide (previously referred to as Unknown B) levels. As it was quantitated using diphenylglycine as a standard rather than by difference (Equation 1), a slight decrease was observed. These results indicate that quantitation by difference is only affected by the variation in the formaldehyde assay.

A summary of the degradation pathway described above is shown in Scheme V as follows.

SCHEME V

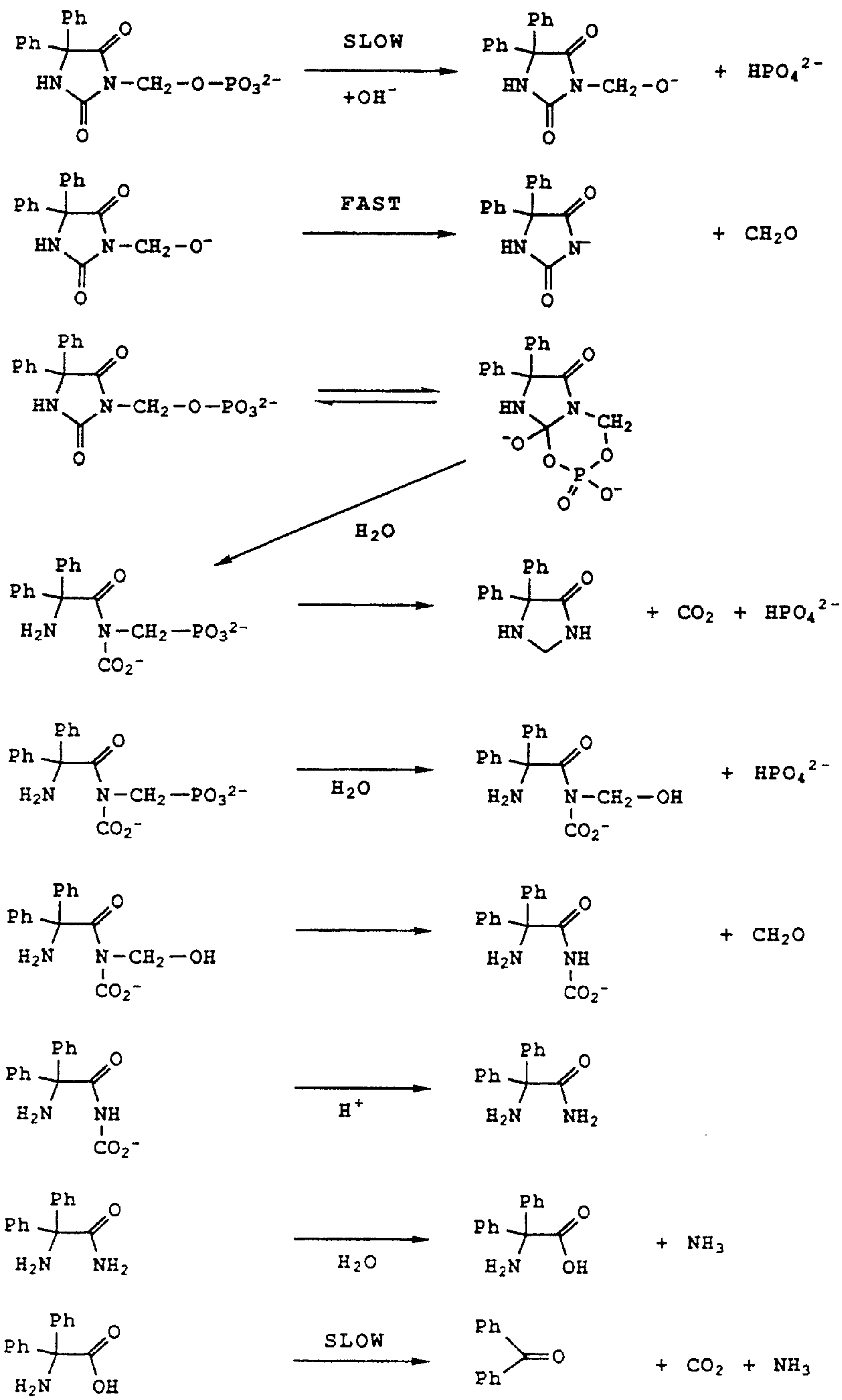

The present invention also provides the preparation of a composition comprising a dosage of a cerebral protective amount.

The present invention also provides the unit dosage form of the formula I or its pharmaceutically acceptable acid addition or base salt, preferably fosphenytoin sodium, is a parenteral formulation, and more particularly, the preferred unit dosage form for formula I and its salts, preferably fosphenytoin sodium, is for administration in intramuscular or intravenous unit dosage form, but also may include an intraarterial form.

The unit dosage form of the formula I and its salts of the present invention, preferably fosphenytoin sodium, may also comprise other compounds useful in the therapy of stroke or common concomitant illness such as in intravenous fluids as well as mannitol or other specific therapies for the concomitant illnesses.

The formulations of the formula I and its salts, although preferably in unit dosage form, may be prepared by any of the methods well known in the art of pharmacy. Such formulations, of course, may include known adjuncts to delay absorption or other agents for a known purpose. In general, the formulations are prepared by solubilizing the formula I and particularly fosphenytoin sodium into an intramuscular or intravenously compatible liquid carrier in a convenient manner.

Supplementary active ingredients can also be incorporated into the formulations of formula I and its salts. The formula I and its salts, and particularly fosphenytoin sodium, can be and is formulated so as to be compatible with the commonly used intravenous fluids including solutions based on dextrose, saline such as 5% or 10% dextrose in water or 0.9% saline as well as more complicated solutions including/such as Ringers lactate, and the like. Unlike available formulations of phenytoin which cause precipitates to form when added to these solutions, formula I and particularly fosphenytoin sodium solutions and mixes with standard intravenous solutions, do not form precipitates and are thus safer and easier to administer.

The preferred unit dosage of the formula I and its salts, and particularly fosphenytoin sodium, is formulated as a near neutral solution (for example, from pH 7 to pH 8.8, preferably pH 8.8), which may include or be added to sterile 5% dextrose or 0.9% saline solution for intravenous or intramuscular injection in concentrations containing the equivalent to 75 mg fosphenytoin sodium per milliliter. Likewise, the stable pharmaceutical composition unit dosage preferably contains about 75 mg fosphenytoin sodium per milliliter. The unit dosage form of formula I or its salts may also contain a preservative such as benzethonium chloride, and the like. The 75 mg/mL solution of formula I or its salts is preferably made more isotonic with sodium chloride.

The usefulness of the compounds of the formula I as well as the stable pharmaceutical composition in the method of the present invention may be demonstrated in various standard test procedures. The following is a description of such a procedure and a showing of the usefulness of fosphenytoin sodium in the present invention method of treating stroke.

The 3-(hydroxymethyl)-5,5-diphenylhydantoin disodium phosphate ester is prepared by the methods described in J. of Pharm. Sci., Vol. 73, No. 8, pp. 1068-73 (August 1984) noted above.

Generally, the procedure shows advantageous effects in a middle cerebral artery occlusion (MCAO) model. The model depends on a surgical procedure which is a modification of that originally proposed by A. Tamura, et al, "Focal Cerebral Ischemia in the Rat: Description of Technique and Early Neuropathological Consequences Following Middle Cerebral Artery Occlusion," J. Cerb. Blood Flow Metab. Vol. 1, pp. 53-60 and is similar to the methods described by Duverger and MacKenzie, "The Quantification of Cerebral Infarction Following Local Ischemia in the Rat: Influence of Strain, Arterial Pressure, Blood Glucose Concentration and Age", J. Cerb. Blood Flow Metab., Vol. 8, pp. 449-461 and S. Brint, et al, "Focal Brain Ischemia in the Rat: Methods for Reproducible Neocortical Infarction Using Tandem Occlusion of the Distal Middle Cerebral and Ipsilateral Common Carotid Arteries", J. Cereb. Blood Flow Metab., Vol. 8, pp. 474-485.

Generally, male F-344 rats weighing 300-350 g are anesthetized in 2% halothane in room air. The right femoral vein is cannulated and the tubing led subcutaneously to an exit behind the neck to allow intravenous (IV) drug injection. The left common carotid is permanently occluded with a 6-0 silk ligature. The left middle cerebral artery (MCA) is exposed through a 2-mm burr hole drilled 1-2 mm rostral to the fusion of the zygomatic arch with the squamosal bone. The dura is cut, the MCA lifted off the surface of the brain, electrocauterized, and cut. The wound margins are sutured shut and the anesthesia stopped.

The stable pharmaceutical composition is used for the assay or a solution for the assay may be prepared as follows: The hydrated fosphenytoin sodium (2.25 $H_2O$ equivalent per mole) is dissolved in saline (pH = 7) at a concentration of 37 mg/mL and administered at 1 mL/kg. This dose of 37 mg/kg is equal to 30 mg/kg of the free base of fosphenytoin sodium. In vivo fosphenytoin sodium or the phenytoin prodrug undergoes a phosphate ester cleavage followed by a dehydroxymethylation to yield the free base of phenytoin. The dose administered is equivalent to 21 mg/kg of the free base of phenytoin.

This solution is then administered intravenously (IV) 30 minutes and 24 hours after occlusion to groups of 12 animals each. Forty-eight hours following occlusion rats are anesthetized with ketamine (150 mg/kg, IP), decapitated, the brains rapidly removed, and placed on ice. With the aid of a brain mold (Activational Systems), the brain is sliced into four 2-mm sections: one section anterior (ANT) to the MCA and three sections posterior (ANT-MED, POST-MED, and POST) to the MCA. The sections are then placed for 30 minutes in a 2% solution of 2,3,5 triphenyltetrazolium chloride (TTC) in saline. The brains are stored in 10% neutral buffered saline for analysis.

The brains are coded and the analysis is performed blinded. The area of infarction in each section is outlined with the aid of a morphometric analysis program (Bioquant IV, R & M Biometrics). The TTC stain is converted into a red marker in live mitochondria, while the infarcted area remains white. The total volume of infarction is calculated from the areas of the four sections assuming three truncated cones. The mean area for each section and the mean total volume are calculated; vehicle and drug treatment are compared using the Student's t-test. The means ± standard deviation, % change, and statistical data are presented in Table I following.

TABLE I

| | Vehicle (N = 12) | Disodium Salt of Prophenytoin (N = 12) | % Change | F value (df = 22) | Probability |
|---|---|---|---|---|---|
| ANT | 11.09 ± 3.95 | 9.03 ± 2.91 | -18.58 | 1.45 | 0.160 |
| ANT-MED | 14.55 ± 4.67 | 11.23 ± 2.95 | -22.82 | 2.08 | 0.049 |
| POS-MED | 12.19 ± 6.39 | 5.23 ± 5.44 | -57.10 | 2.87 | 0.008 |
| POST | 10.44 ± 7.38 | 2.97 ± 4.39 | -71.56 | 3.01 | 0.006 |
| TOTAL VOLUME | 74.59 ±32.16 | 43.21 ±21.85 | -42.07 | 2.80 | 0.010 |

If the human is at risk of stroke or suffering from a stroke, the compound of formula I or an equivalent amount of the composition prodrug in the stable pharmaceutical composition may be administered before or preferably as soon as possible after the onset of a stroke to within about 24 hours after the onset of brain or spinal cord ischemia or stroke in either an initial intravenous (IV) or intramuscular (IM) dose in a range of 15 mg/kg to 37.5 mg/kg, preferably 30 mg/kg which may be and preferably is followed by a repetition of a smaller dose (7.5 to 15 mg/kg) daily for up to 14 days.

Generally the compound of formula I or an equivalent amount of the composition prodrug in the stable pharmaceutical composition may be administered initially in the one larger dose followed by the smaller doses at regular intervals of about 12 to 24 hours after the initial dose. However, the administration may be maintained by a slow intravenous drip if the physician deemed such administration to be preferred for a particular condition.

The above description is a preferred embodiment and, therefore, to be construed as illustrative. It is not meant to be limiting to utilization of the present invention to its fullest extent and variations to the description within the ordinary skill in the art are also meant to be included in the invention.

## Claims

1. A method of use of a compound of the formula (I)

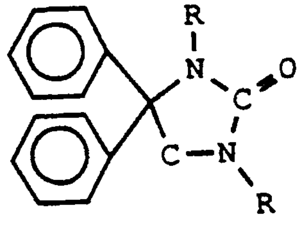

I

or the pharmaceutically acceptable acid addition or basic salt, $C_1$-$C_4$ alkylhalide quaternary salt or N-oxide thereof;

wherein each R is hydrogen or $-CH(R_1)-X-P(O)(OH)_2$ wherein $R_1$ is selected from the group consisting of hydrogen or $C_1$-$C_7$ straight or branched chain alkyl; with the proviso that the R's cannot simultaneously be hydrogen for the preparation of pharmaceutical compositions for treating cerebral ischemia or stroke.

2. A method of Claim 1 wherein the compound is 3-(hydroxymethyl)-5,5-diphenylhydantoin phosphate ester.

3. A method of Claim 2 wherein the compound is the fosphenytoin sodium or the disodium salt of 3-(hydroxymethyl)-5,5-diphenylhydantoin phosphate ester.

4. A method of Claim 1 wherein the unit dosage form of said pharmaceutical composition comprises of about 75 mg/milliliter of the free base of the 3-(hydroxymethyl)-5,5-diphenylhydantoin phosphate ester or equivalent thereof.

5. A method of preparing a pharmaceutical composition for the treatment of cerebral ischemia or a stroke in a mammal which may be administered by the subcutaneous, intramuscular, or intravenous route to a mammal, by dissolving 3-(hydroxymethyl)-5,5-diphenylhydantoin disodium phosphase ester in 35 to 130 mg/mL and 0.05 to 2 M of a buffer effective to maintain a pH of about 8.3 to 9.4.